# EUROPEAN PATENT SPECIFICATION

(11) **EP 2 237 755 B1**
(45) Date of publication and mention of the grant of the patent: **18.06.2014**
(21) Application number: 08855752.5
(22) Date of filing: 28.11.2008
(51) Int. Cl.: A61F 13/02, A61M 1/00, A61M 27/00

(54) **WOUND PACKING MEMBERS**
WUNDPACKELEMENTE
ÉLÉMENTS DE PANSEMENT POUR PLAIES

(30) Priority: 08.12.2007 GB 0724040
(43) Date of publication of application: 13.10.2010
(73) Proprietor: Smith & Nephew PLC, London WC2N 6LA (GB)
(72) Inventor: HALL, Kristian, Hull HU13 0HN (GB)
(74) Representative: Connors, Martin L.H.
(86) International application number: PCT/GB2008/051134
(87) International publication number: WO 2009/071938

(56) References cited:
- WO-A-95/03018
- WO-A-2006/089551
- DE-U1-202004 018 245

## Description

The present invention relates to members for filling a volume of a wound particularly, though not exclusively, during topical negative pressure (TNP) therapy.

In recent years TNP therapy had become increasingly important in the field of improved treatment of wounds by making the healing thereof faster and more controlled.

The basic principle of TNP therapy is to create a closed cavity over the wound itself by means of a thin, flexible sealing film adhered to the patient's sound skin surrounding the wound; admitting one end of an aspirant conduit into the closed cavity, the conduit being sealed to the flexible film, for example; and connecting a distal end of the aspirant conduit to a vacuum source such as an electrically driven vacuum pump, for example, to create a pressure lower than the surrounding ambient atmospheric pressure within the wound cavity. As is known to the skilled person the lower pressure creates many beneficial therapeutic effects on the wound including increased blood flow to the wound and faster formation of granulation tissue, for example. There are very many variations on this basic principle of TNP therapy.

The types of wounds treated by TNP therapy generally range from quite small at about 5cm² to very large traumatic wounds and burns of no particular maximum dimension. Such wounds may also have significant depth and therefore, significant volume. It is necessary to control the way in which a wound heals. For example, the wound should heal from the base up and close in from the edges desirably in a uniform manner. In particular it is desirable that the wound does not close over and form an occluded cavity in the flesh which is extremely undesirable from the patient's point of view as such form sites for infection.

To prevent the formation of occluded cavities during TNP therapy, the wound is usually packed with a filler which desirably has some resilience or "bounce" to resist the compressive forces created during TNP therapy by outside ambient atmospheric pressure bearing down on the wound due to the vacuum created in the wound cavity. The purpose of the filler is to keep the surrounding edges of the wound apart so that they cannot grow over and form a cavity. The filler may also perform the function of providing fluid flow channels between the wound and the filler or through the filler in order to provide a uniform reduced pressure distribution over the surface area of the wound and to promote efficient aspiration of exudate fluids away from the wound surface and generally into a remote waste receptacle associated with the aspirant conduit. An example of such fillers is shown in DE 20 2004 0182 45 U1. As noted above there are very many variations on the basic TNP therapy principle and to illustrate how complex TNP therapy may be reference is made to the documents described below and which are of common ownership herewith.

In our co-pending International patent application, WO 2004/037334, apparatus, a wound dressing and a method for aspirating, irrigating and cleansing wounds are described. In very general terms, this invention describes the treatment of a wound by the application of topical negative pressure (TNP) therapy for aspirating the wound together with the further provision of additional fluid for irrigating and/or cleansing the wound, which fluid, comprising both wound exudates and irrigation fluid, is then drawn off by the aspiration means and circulated through means for separating the beneficial materials therein from deleterious materials. The materials which are beneficial to wound healing are recirculated through the wound dressing and those materials deleterious to wound healing are discarded to a waste collection bag or vessel.

In our co-pending International patent application, WO 2005/04670, apparatus, a wound dressing and a method for cleansing a wound using aspiration, irrigation and cleansing wounds are described. Again, in very general terms, the invention described in this document utilises similar apparatus to that in WO 2004/037334 with regard to the aspiration, irrigation and cleansing of the wound, however, it further includes the important additional step of providing heating means to control the temperature of that beneficial material being returned to the wound site/dressing so that it is at an optimum temperature, for example, to have the most efficacious therapeutic effect on the wound.

In our co-pending International patent application, WO 2005/105180, apparatus and a method for the aspiration, irrigation and/or cleansing of wounds are described. Again, in very general terms, this document describes similar apparatus to the two previously mentioned documents hereinabove but with the additional step of providing means for the supply and application of physiologically active agents to the wound site/dressing to promote wound healing.

In spite of the self evident growing complexity of TNP therapy in general the field of wound fillers, which are a vital element in the therapy has changed or improved very little over the years that TNP therapy has been developing. Aside from complicated and expensive inflatable bags, most of the fillers in use are based either on foam or on cotton gauze. Foam fillers are usually cut with scissors to the required shape (of the wound) by a clinician. However both foams and gauzes have the disadvantage that the cell or pore size is often too large and often results in growing tissue growing into the cells and adhering the foam to the wound causing further damage and trauma to the wound and patient on removal. When gauze is used as a filler, however, clinicians are instructed to "fluff up" the gauze to increase its volume which can cause problems in that the actual form of the gauze as packed into wounds is very variable. A further disadvantage with both foams and gauze when cut to fit wounds is that of debris. Gauze in particular is prone to shedding fibres and particles and when cut, these inevitably find their way into the wound and become occluded therein which can lead to later infection.

It is an object of the present invention to overcome or mitigate some of the disadvantages of known wound fillers.

According to a first aspect of the present invention there is provided a wound packing unit comprising a resilient, fluid absorbent material contained within a porous bag member, the porous bag member being made of a material which is non-adherent to the wound.

The resilient, fluid absorbent material may be any suitable known material such as gauze, foams and the like which are known for use in wound packing.

The porous bag member may be any suitable material known to be non-adherent to a wound and especially to new growing tissue. As noted above, one of the problems of using known gauzes and foams for wound packing or filling materials is that the pore sizes of the material often leads to in-growth of granulation tissue into the pores and which may be damaged on changing a wound dressing and also causing pain to the patient. The size of surface porosity of the bag member may be such that growing tissue does not grow into the surface pores and thus does not create a mechanical connection thereto. The material from which the porous bag member may be formed may be a suitable plastics sheet or net material such as EVA, PU, PP, PE, silicone, carbomethoxy cellulose, polyacrylate, for example.

The plastics sheet may comprise nets, woven materials, non-woven fibrous sheet, foams, electrospun nano fibres, for example, from a wide variety of different materials such as those noted above, for example.

A required porosity may be endowed in the case of sheet film material by perforating the sheet with any desired pattern or form of perforations.

The wound packing unit may be made from two sheets of suitable material having substantially coterminous outer shapes, for example, and be welded together at their outer peripheries so as to contain the resilient absorbent material in the cavity so formed. The bag member may comprise two sheets of material joined at their peripheries to form a pocket therein to receive the resilient, fluid absorbing material therein.

In one embodiment the sheets from which the wound packing units are made may be circular, for example.

The wound packing unit according to the present invention may be used as a general, stand-alone wound filling medium in the context of a dressing used for the treatment of any type of wound. Furthermore, the wound filling unit as defined is not used in a subdivided form by, for example, cutting into a smaller member. A particular advantage of using the wound packing units as one or more units to fill a wound without cutting as with known wound packing materials such as gauze and foam on their own is that there is no shedding of particles due to cutting and consequently no occluded particles in the wound which may lead to infection.

Whilst the wound packing unit may be used singly or in a plurality thereof as general wound filling member(s) in any dressing where suitable, the wound packing unit according to the present invention may advantageously be used as part of a wound dressing in TNP therapy. In such a dressing, the wound packing unit or plurality of them may be used to pack the wound and, the most basic form of TNP therapy, a covering film or drape adhered over the wound to the patient's sound flesh surrounding the wound in order to form a closed or sealed cavity over the wound, and an aspirant conduit connected to a vacuum source being admitted to the so-formed wound cavity.

When used as part of a dressing in TNP therapy the sheet material may desirably be textured so as to form channels in the surface adjacent the wound surface to further enhance the generation of an even negative pressure distribution over the surface of the wound and to enhance the flow and drainage of wound exudate to an aspirant conduit so that it may be aspirated from the wound vicinity into a remotely located waste receptacle. Whilst the filling of the bag member may be an absorbent material able to absorb wound exudate, it is nevertheless beneficial that as much of the exudate as possible is aspirated to a waste receptacle. In this way risk of infection due to build up of exudate may be minimised.

In one embodiment of a porous bag member according to the present invention the textured surface may comprise an array of hexagonal indentations or depressions in the bag material surface effectively producing a three-dimensional surface structure, each indentation or depression having a perforation at its centre to allow fluid flow, both gaseous and liquid, over both surfaces of the bag member, i.e. between the wound surface and bag surface and between the bag surface and filling. A suitable material from which the bag member may be made may be a vacuum formed plastics sheet material such as, for example, EVA film which is soft and non-adherent to a wound surface.

The bag member may have any desired surface topography and formations which are conducive to wound surface therapy.

When used in TNP therapy, the bag member may have an aspirant conduit having a drain portion affixed during manufacture with its drain end inside the bag member and surrounded by the resilient absorbent filling. In this way time may be saved applying a TNP dressing and also the correct positioning of the aspirant conduit may be ensured.

The resilient absorbent filling material within the bag member may also be provided with antimicrobial additives such as silver, for example, to reduce or control bacterial load so as to control infection.

The resilient absorbent material filling of the wound packing unit may also advantageously be provided with other biologically active components in addition to or in substitution of silver, such as antimicrobial compounds e.g. silver containing species, iodine, guanidines, biguanidines etc., analgesics e.g. aspirin, ibuprofen etc., anaesthetics e.g. amethocaine, lignocaine etc., growth factors e.g. VEGF PDGF, vasodilators e.g. NO, sildenafil, histamine etc. for example. Other examples of suitable active components may be found in WO 2005/105180.

The sheet material of the wound packing units according to the present invention may optionally be soaked or coated in a non-adherent gel such as, for example, a petroleum gel an example of which is Cuticerin (trade mark), silicone gel or a hydrogel.

As with the filling the sheet material forming the porous bag member may also be treated with silver for antimicrobial purposes etc.

The resilient, fluid absorbent material within the porous bag member or the porous bag member itself may be formed from laminated material so as to provide a texture to the wound packing member unit. Such laminated material may have a "quilted" type of texture where two layers of material may be welded together in a pre-determined pattern with a layer of a resilient material such as an open cell polymer foam, for example, between. The two outer material layers of the laminate may be provided with an array of pores in predetermined fashion to permit ingress of exudate. Some cells formed by a laminating procedure may be left unperforated so as to provide resilience by virtue of compressing the air trapped inside unperforated pockets or cells so formed.

A porous bag member may be formed from two layers of laminated material as described above. Furthermore, a wound packing unit so formed may also further comprise resilient, fluid absorbent material therebetween.

The types of sealing film drapes generally used to form the overlying wound sealing medium in TNP dressings usually have a coating of adhesive over the whole surface of the film. Therefore, it may be desirable to place a second sheet of non-adhesively coated film between the sealing film drape and the wound packing unit of the present invention so that when the sealing drape is removed as the first step of a dressing change the wound packing unit is not torn from the wound with it.

According to a second aspect of the present invention there is provided a method for packing a wound cavity comprising the steps of; forming a wound packing unit by enclosing a resilient absorbent material within a porous bag member, the porous bag member being made of a material which is non-adherent to the wound.

In order that the present invention may be more fully understood examples will now be described by way of illustration only with reference to the accompanying drawings, of which:
Figures 1A and 1B show a cross section through a bag member of a wound packing unit made from embossed EVA material and a detail thereof, respectively;
Figures 2A and 2B show a plan view of the embossed pattern of the material of the bag member of Figure 1 and a cross section therethrough, respectively;
Figure 3 shows a photograph of two examples of wound packing units according to the present invention;
Figure 4 shows a schematic cross section of one embodiment of a wound packing unit incorporated into a TNP therapy dressing; and
Figure 5 which shows a second embodiment of a wound packing unit incorporated into a TNP therapy dressing.

Referring now to the drawings and where the same features are denoted by common reference numerals.

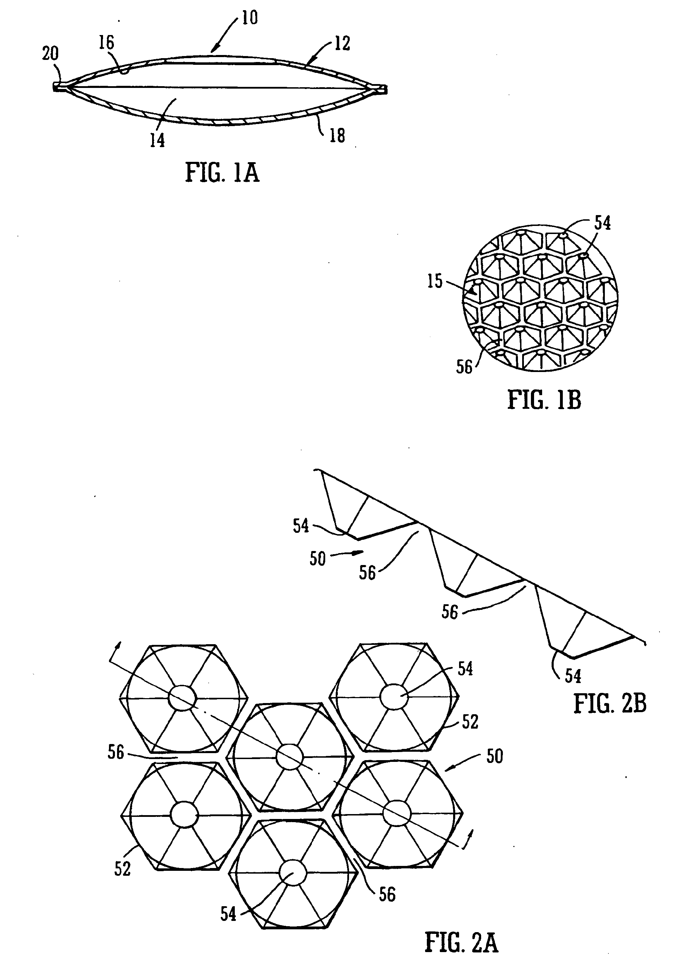

Figure 1 shows a cross section through a wound packing unit 10 comprising a bag member 12 and a filing 14 of a resilient, fluid absorbent material. The bag member is made from thin, embossed EVA material sheet 15, a small enlarged area of which is shown at Figure 1B, and comprises two circular sheet portions 16, 18 which are RF welded 20 around their peripheries. The two sheets are partially welded around their peripheries, filled with the suitable resilient and absorbent filing material 14 such as gauze, foam, chipped foam or non woven, and then the remainder of the periphery is welded so as to form a completely sealed wound packing unit apart from the porosity of the sheets 16, 18 themselves. Thus, the wound packing unit 10 also forms a resilient and fluid absorbing medium with which to pack wounds being treated.

The bag member 12 is manufactured from a thin flexible plastics material such as EVA, for example and which has a vacuum-formed textured surface 50. The surface topography comprises an array of hexagons 52 having a central perforation 54 in each hexagon (although not all indentations may be perforated). The individual hexagons are separated by channels 56 as shown in Figures 2A and 2B. Figure 2B shows the contour of the hexagonal indentations from a side view. In the illustrated embodiment the hexagons 52 are 2.6 mm across the flats, the central perforations 54 are 0.6mm in diameter and the depth of the hexagonal indentations from the planar surface is 1.05mm. However, these dimensions are merely exemplary of one embodiment of a bag member according to the present invention and dimensions may vary according to the needs of a particular wound in other embodiments.

Although the example of the wound filling device described above is stated to be made with circular sheets of material, it is of course merely exemplary and the bag 12 may be made to any required shape and size to suit a particular wound shape and size or a range of wound shapes and sizes.

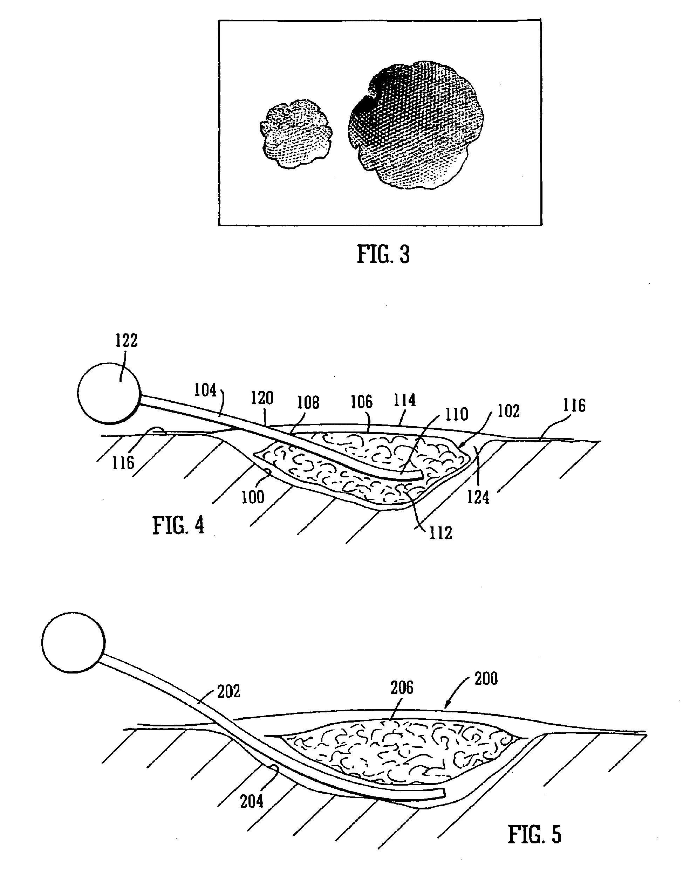

The wound packing unit described above may be used to fill any general wound and Figure 3 shows two examples of wound packing units; the larger one being approximately 10 cm in diameter and the smaller one approximately 5 cm in diameter.

Figure 4 shows a schematic cross section through a wound 100 being treated with TNP therapy. The wound 100 has a wound packing unit 102 as described with reference to Figures 1 and 2 and is provided as an entity complete with an aspirant conduit 104 which is incorporated in and sealed to the bag member 106 at 108 where it passes through. The end drain portion 110 of the conduit 104 is embedded in and wrapped with the filling material 112 of the wound packing unit. A sealing drape 114 is adhered to the patient's sound flesh 116 around the border of the wound 100. The sealing drape 114 is provided with a coating of pressure sensitive adhesive (not shown) over the surface which is to be adhered to the patient and the conduit 104 where it passes through the sealing drape is sealed to the drape by means of the drape being pinched around the conduit itself at 120. The conduit 104 is connected to a vacuum source 122 such as an electric vacuum pump, for example. The vacuum source may also be associated with a waste receptacle (not shown) in known manner and to which wound exudate from the wound 100 may be aspirated. The closed volume beneath the sealing drape 114 forms a wound cavity 124 which is subject to a negative pressure as created by the vacuum source 122. Outside ambient atmospheric pressure bears down upon the wound packing unit 102 so as to compress it and hold the sides/edges of the wound apart to prevent them growing over and forming an occluded cavity in the wound.

The embodiment 200 shown in Figure 5 is similar to that of Figure 4 except that the conduit 202 is placed in the wound 204 separately from the wound packing unit 206. In essence this embodiment works in a similar manner to that of Figure 4.

Both the embodiments of Figures 4 and 5 work in essentially the same manner in that the vacuum created in the wound cavity is evenly distributed by virtue of the perforations and channels in the embossed material forming the bag member thus, allowing fluid access to and from all parts of the wound surface. The perforations and channels allow transport of wound exudate both over the wound surface and also through the interior of the wound packing unit towards the aspirant conduit in each case.

Throughout the description and claims of this specification, the words "comprise" and "contain" and variations of the words, for example "comprising" and "comprises", means "including but not limited to", and is not intended to (and does not) exclude other moieties, additives, components, integers or steps.

## Claims

1. A wound packing unit (10) comprising a resilient, fluid absorbent material (14) contained within a porous bag member (12), the porous bag member being made of a material which is non-adherent to the wound (100) and the porous bag member is formed from a laminated material **characterised in that** there is an array of hexagonal indentations or depressions in the bag material surface effectively producing a three dimensional surface structure, each indentation or depression having a perforation at its centre.

2. A wound packing unit (10) according to claim 1 wherein the resilient, fluid absorbent material (14) is selected from the group comprising: gauze; foams and non woven material.

3. A wound packing unit (10) according to claim 1 wherein the material from which an outer surface of the porous bag member is formed is selected from the group comprising: EVA, PU, PP, PE, silicone, carbomethoxy cellulose and polyacrylate.

4. A wound packing unit (10) as claimed in any one preceding claim wherein the bag member (12) comprises two sheets (16,18) of material joined at their peripheries to form a pocket therein to receive the resilient, fluid absorbing material (14) therein.

5. A wound packing unit (10) according to claim 4 wherein the sheet material (15) is coated in silver.

6. A wound packing unit (10) according to any one preceding claim wherein the resilient, fluid absorbent material (14) is treated with silver.

7. A wound packing unit (10) according to any one preceding claim wherein the resilient, fluid absorbent material (14) is treated with biologically active components.

8. A wound packing unit (10) as claimed in any one preceding claim further having combined therewith a conduit (104) for use in topical negative pressure therapy.

9. A wound packing unit (10) according to claim 4 wherein the sheet material (15) is selected from the group comprising: perforated and embossed sheet, woven materials, non-woven fibrous sheet, foams and electrospun nano fibres.

10. A wound packing unit (10) according to any one preceding claim wherein the laminated material comprises a foam material between two outer layers.

11. A wound packing unit (10) according to claim 10 wherein the cells or pockets are formed by welding together of the two outer layers.

12. A wound packing unit (10) according to claim 10 or claim 11 wherein the bag material has a quilted texture.

## Patentansprüche

1. Eine Wundpackeinheit (10), die ein elastisches, flüssigkeitsaufsaugendes Material (14) beinhaltet, das in einem porigen Taschenelement (12) enthalten ist, wobei das porige Taschenelement aus einem Material gemacht ist, das nicht an der Wunde (100) haftet, und das porige Taschenelement aus einem laminierten Material gebildet ist, **dadurch gekennzeichnet, dass** es eine Anordnung von sechseckigen Vertiefungen oder Einbuchtungen in der Taschenmaterialoberfläche gibt, die wirksam eine dreidimensionale Oberflächenstruktur erzeugt, wobei jede Vertiefung oder Einbuchtung in ihrer Mitte eine Perforierung aufweist.

2. Wundpackeinheit (10) gemäß Anspruch 1, wobei das elastische, flüssigkeitsaufsaugende Material (14) ausgewählt ist aus der Gruppe, die beinhaltet: Gaze, Schäume und Vliesstoff.

3. Wundpackeinheit (10) gemäß Anspruch 1, wobei das Material, aus dem eine äußere Oberfläche des porigen Taschenelements gebildet ist, ausgewählt ist aus der Gruppe, die beinhaltet: EVA, PU, PP, PE, Silikon, Carbomethoxycellulose und Polyacrylat.

4. Wundpackeinheit (10) gemäß einem der vorhergehenden Ansprüche, wobei das Taschenelement (12) zwei Lagen (16, 18) aus Material beinhaltet, die an ihren Randbereichen zusammengefügt sind, um ein Fach darin zu bilden, um das elastische, flüssigkeitsaufsaugende Material (14) darin aufzunehmen.

5. Wundpackeinheit (10) gemäß Anspruch 4, wobei das Lagenmaterial (15) mit Silber beschichtet ist.

6. Wundpackeinheit (10) gemäß einem der vorhergehenden Ansprüche, wobei das elastische, flüssigkeitsaufsaugende Material (14) mit Silber behandelt ist.

7. Wundpackeinheit (10) gemäß einem der vorhergehenden Ansprüche, wobei das elastische, flüssigkeitsaufsaugende Material (14) mit biologisch aktiven Komponenten behandelt ist.

8. Wundpackeinheit (10) gemäß einem der vorhergehenden Ansprüche, die weiter eine mit ihr kombinierte Leitung (104) zur Verwendung in der lokalen Unterdrucktherapie aufweist.

9. Wundpackeinheit (10) gemäß Anspruch 4, wobei das Lagenmaterial (15) ausgewählt ist aus der Gruppe, die beinhaltet: perforierte und geprägte Lage, gewebte Materialien, Faservlieslage, Schäume und elektrogesponnene Nanofasern.

10. Wundpackeinheit (10) gemäß einem der vorhergehenden Ansprüche, wobei das laminierte Material zwischen zwei äußeren Schichten ein Schaummaterial beinhaltet.

11. Wundpackeinheit (10) gemäß Anspruch 10, wobei die Zellen oder Fächer durch das Zusammenschweißen der zwei äußeren Schichten gebildet werden.

12. Wundpackeinheit (10) gemäß Anspruch 10 oder Anspruch 11, wobei das Taschenmaterial eine gesteppte Beschaffenheit aufweist.

## Revendications

1. Une unité de compresse pour plaie (10) comprenant un matériau résilient absorbant les fluides (14) contenu à l'intérieur d'un élément formant sac poreux (12), l'élément formant sac poreux étant fait d'un matériau qui n'adhère pas à la plaie (100) et l'élément formant sac poreux est formé à partir d'un matériau laminé **caractérisé en ce qu'**il y a un ensemble de dentelures ou de dépressions hexagonales dans la surface de matériau de sac produisant en réalité une structure de surface tridimensionnelle, chaque dentelure ou dépression ayant une perforation au niveau de son centre.

2. Une unité de compresse pour plaie (10) selon la revendication 1 dans laquelle le matériau résilient absorbant les fluides (14) est sélectionné dans le groupe composé de : gaze ; mousses et matériau non-tissé.

3. Une unité de compresse pour plaie (10) selon la revendication 1 dans laquelle le matériau à partir duquel une surface externe de l'élément formant sac poreux est formée est sélectionné dans le groupe composé de : EVA, PU, PP, PE, silicone, carbométhoxy cellulose et polyacrylate.

4. Une unité de compresse pour plaie (10) telle que revendiquée dans n'importe quelle revendication précédente dans laquelle l'élément formant sac (12) comprend deux feuilles (16, 18) de matériau jointes au niveau de leurs périphéries pour former une poche à l'intérieur afin de recevoir le matériau résilient absorbant les fluides (14) à l'intérieur.

5. Une unité de compresse pour plaie (10) selon la revendication 4 dans laquelle le matériau de feuille (15) est revêtu d'argent.

6. Une unité de compresse pour plaie (10) selon n'importe quelle revendication précédente dans laquelle le matériau résilient absorbant les fluides (14) est traité avec de l'argent.

7. Une unité de compresse pour plaie (10) selon n'importe quelle revendication précédente dans laquelle le matériau résilient absorbant les fluides (14) est traité avec des composants biologiquement actifs.

8. Une unité de compresse pour plaie (10) telle que revendiquée dans n'importe quelle revendication précédente à laquelle est en outre combiné un conduit (104) pour une utilisation en thérapie par pression négative topique.

9. Une unité de compresse pour plaie (10) selon la revendication 4 dans laquelle le matériau de feuille (15) est sélectionné dans le groupe composé de : feuille perforée et grainée, matériaux tissés, feuille fibreuse non-tissée, mousses et nano-fibres électrofilées.

10. Une unité de compresse pour plaie (10) selon n'importe quelle revendication précédente dans laquelle le matériau laminé comprend un matériau en mousse entre deux couches externes.

11. Une unité de compresse pour plaie (10) selon la revendication 10 dans laquelle les cellules ou poches sont formées en soudant ensemble les deux couches externes.

12. Une unité de compresse pour plaie (10) selon la revendication 10 ou la revendication 11 dans laquelle le matériau de sac a une texture matelassée.
